# EUROPEAN PATENT SPECIFICATION

(11) **EP 3 666 264 B1**
(45) Date of publication and mention of the grant of the patent: **07.06.2023**
(21) Application number: 16876109.6
(22) Date of filing: 19.12.2016
(51) Int. Cl.: A61K 9/16, A61K 38/28, A61P 3/10

(54) **BIODEGRADABLE COVALENT MATRICES FOR THE ORAL DELIVERY OF INSULIN DIRECTED TO THE COLON, ACTIVATED BY MICROBIOTA, AND PRODUCTION METHOD THEREOF**
BIOLOGISCH ABBAUBARE KOVALENTE MATRIZEN ZUR ORALEN VERABREICHUNG VON INSULIN, DAS AN DEN DARM GERICHTET IST, AKTIVIERT DURCH MIKROBIOTA, UND HERSTELLUNGSVERFAHREN DAFÜR
MATRICES COVALENTES BIODÉGRADABLES POUR L'ADMINISTRATION D'INSULINE PAR VOIE ORALE DESTINÉE AU CÔLON À ACTIVATION PAR LE MICROBIOTE ET LEUR PROCÉDÉ D'OBTENTION

(30) Priority: 18.12.2015 MX 2015017857
(43) Date of publication of application: 17.06.2020
(73) Proprietor: Centro De Investigación En Alimentación Y Desarrollo, A.C., Hermosillo, Sonora 83304 (MX)
(72) Inventor: MARTÍNEZ-LÓPEZ, Ana Luisa, Hermosillo Sonora 83304 (MX); CARVAJAL-MILLÁN, Elizabeth, Hermosillo Sonora 83304 (MX); SOTELO-CRUZ, Norberto, Hermosillo Sonora 83190 (MX); MICARD, Valerie, 34060 Montpellier (FR); RASCÓN CHU, Agustín, Hermosillo Sonora 83304 (MX); PRAKASH, Satya, Montreal Québec 3775 (CA); LIZARDI-MENDOZA, Jaime, Hermosillo Sonora 83304 (MX); LÓPEZ-FRANCO, Yolanda Leticia, Hermosillo Sonora 83304 (MX); CANETT-ROMERO, Rafael, Hermosillo Sonora 83249 (MX); TOLEDO-GUILLÉN, Alma Rosa, Hermosillo Sonora 83304 (MX)
(74) Representative: M. Zardi & Co S.A.
(86) International application number: PCT/MX2016/000170
(87) International publication number: WO 2017/105212

(56) References cited:
- WO-A2-01/49320
- BERLANGA-REYES C.M. ET AL.: "Maize Arabinoxylan Gels as Protein Delivery Matrices", MOLECULES, vol. 14, no. 4, 8 April 2009 (2009-04-08), pages 1475-1482, XP055437409, DOI: 10.3390/molecules14041475
- MARTÍNEZ-LÓPEZ A.L. ET AL.: "Arabinoxylan Microspheres: Structural and Textural Characteristics", MOLECULES, vol. 18, no. 4, 19 April 2013 (2013-04-19) , pages 4640-4650, XP055773584, DE ISSN: 1433-1373, DOI: 10.3390/molecules18044640
- BERLANGA-REYES, CLAUDIA M. ET AL.: 'Maize arabinoxilan gels as protein delivery matrices' MOLECULES, [Online] vol. 14, no. 4, 08 April 2009, pages 1475 - 1482, XP055437409 Retrieved from the Internet: <URL:www.mdpi.com/journal/molecules> [retrieved on 2017-12-07]
- GUEVARA-BRETON, N. A. ET AL.: 'encapsulación: técnicas and aplicaciones in the industria alimentaria' TEMAS SELECTOS OF INGENIERíA OF ALIMENTOS vol. 2, 2008, pages 36 - 49, XP055437412
- NINO-MEDINA GUILLERMO ET AL.: 'Feruloylated arabinoxylans and arabinoxylan gels: structure, sources and applications' PHYTOCHEMISTRY REVIEWS MAR 2010 vol. 9, no. 1, 28 February 2010, ISSN 1568-7767 pages 111 - 120, XP019794059
- KUMAR VIKAS ET AL.: 'Dietary Roles of Non-Starch Polysachharides in Human Nutrition: A Review' CRITICAL REVIEWS IN FOOD SCIENCE AND NUTRITION vol. 52, no. 10, October 2011, ISSN 1040-8398 pages 899 - 935, XP055437415
- MOZHAEV, VADIM V. ET AL.: 'Catalytic activity and denaturation of enzymes in waterlorganic cosolvent mixtures' EUR. J. BIOCHEM. vol. 184, February 1989, pages 597 - 602, XP055437419

## Description

### BACKGROUND OF THE INVENTION

### Field of the Invention

This invention relates to a covalent biomaterial formed with gel-forming feruloylated polysaccharides, which is biodegradable and allows oral insulin administration targeting the colon. This invention also includes a method for the preparation of covalently cross-linked particles for use enzymatically in the biomedical or pharmaceutical industry.

### Description of the Related Technology

Insulin is a hormone required in the treatment of patients with diabetes mellitus, but its sub-cutaneous route for administration is a painful and invasive therapy; additionally, it may cause side effects such as hypoglycemia, allergies, and lipodystrophy (Rekha y Sharma, 2013). For this reason, strategies for alternate and non-invasive routes of administration of this therapeutic peptide are being sought worldwide. Oral administration of insulin has the greatest potential because the specific delivery into the colon presents interesting characteristics such as a long residence time, decreased proteolytic activity and natural absorption characteristics (Yang et al., 2002). Nevertheless, successful delivery of insulin via this route requires that biological barriers be overcome, such as enzymatic degradation while passing through the upper gastrointestinal tract (UGI, stomach and small intestine), and to remain biologically active when it arrives at the specific absorption sites. To overcome these barriers, investigations have focused on designing matrices allowing the encapsulation and controlled release of insulin into the colon.

Encapsulation of insulin in matrices based polysaccharides, has been extensively studied in recent years as a strategy for the development of systems for the controlled release of oral insulin (Owens, 2002; Khafagy et al., 2007). Furthermore, polysaccharides are susceptible to degradation by the microbiota, a characteristic which allows the development of delivery systems specific to the colon (Gibson et al., 2004). In the Prior Art, there are publications citing polysaccharides used for the development of encapsulation systems and insulin release in the colon, for example chitosan, pectin, guar gum, inulin and dextrans, among others (Liu et al., 2008). However, most of these strategies have the disadvantage of its limited stability in certain conditions of pH and ionic strength, therefore they are chemically modified or combined with hydrophobic polymers (Coviello et al., 2007). Moreover, many require the use of organic solvents and high temperatures, aspects which greatly limit the use of these systems.

An alternative to these systems is the use of polysaccharides with the ability to form covalent gels enzymatically. Unlike most polysaccharides, feruloylated arabinoxylans (FAX) is capable of forming a covalent matrix enzymatically, which is a characteristic that allows them to be irreversible (Izydorczyk and Biliaderis, 1995). Because of this, temperature changes, ionic strength and pH have little effect on them (Carvajal-Millan et al., 2005, 2006). Covalent gels are generally strong, they are formed quickly and are not degraded by the upper gastrointestinal system. Based on the above, in the last 10 years the use of FAX has only been reported for models for trapping molecules such as proteins (Carvajal-Millan et al., 2006), probiotics (Morales-Ortega et al., 2014), lycopene (Hernandez-Espinoza et al., 2012), diclofenac (Iqbal et al., 2011) and methyl-xanthine (Iravani et al., 2011). These investigations were focused on determining the effect of the gel structure with the ability to trap different molecules.

Article Maize Arabinoxylan Gels as Protein Delivery Matrices of Claudia M. Berlanga-Reyes et al. (2009) discloses crosslinked maize arabinoxylan gels entrapping insulin as delivery matrix where the gel degradation is caused by colonic bacteria. However, such feruloylated arabinoxylan matrix is degraded in the acidic digestive tract and the insulin that reach the colon is not bio-disponible.

Furthermore, trapping on particles (beads, microspheres, microcapsules) is the most frequently reported for the release of bioactive compounds for oral administration, due to the advantages during the dosing process and its semi-automated manufacturing. Until the development of this invention, there was no information available regarding the encapsulation of biologically active insulin on FAX particles for targeted release in the colon. This is because the available methodologies for making particles with other polysaccharides are based on ionic gelation, the combination with synthetic or hydrophobic polymers or by polymer precipitation. However, FAXs are neutral and hydrophilic polysaccharides which cannot be formed by the aforementioned techniques. Therefore, the inventors have developed a process to encapsulate insulin in FAX particles obtained by a process of enzymatic gelation, which allows efficient entrapment of biologically active insulin and its subsequent release in the colon.

### SUMMARY OF THE INVENTION

The object of this invention consists in forming a covalent biomaterial from gel-forming feruloylated polysaccharides, which is biodegradable and makes it possible to orally administer insulin to be directed to the colon. This invention also includes a method to prepare covalently cross-linked particles enzymatically.

This is achieved by a process for the formation of a covalent matrix from feruloylated arabinoxylans gelling agents carrying insulin to the colon, said matrix having a particle formation, that includes: (a) preparing an aqueous solution of feruloylated arabinoxylans, from feruloylated arabinoxylans with gelling capacity; that has an esterified ferulic acid to arabinoxylans content of between 0.02 and 22 µg/mg; (b) preparing an aqueous solution of an enzymatic crosslinking agent; (c) preparing an aqueous solution of insulin or its analogues aggregated with glutamic acid; and (d) producing insulin or insulin analogue-containing particles covalently crosslinked by electrospray or manual extrusion on a hydrophobic bed.

### BRIEF DESCRIPTION OF THE DRAWINGS

Figura 1A is an image obtained by optical microscopy, and Figure 1B is an image obtained by Scanning electron microscopy of the covalent microsphere of the FAX with insulin dehydrated by freeze drying. It is shown the form and size of the microsphere in Figure1B.
Figure 2 is an image obtained by laser scanning confocal microscopy of the focal insulin distribution in the covalent microsphere of the FAX. The insulin was rhodamine stained to observe its distribution and aggregate sizes.
Figure 3A, 3B, 3C and 3D are images obtained by optical microscopy of the FAX covalent microcapsule recovered from an *in vitro* human gastrointestinal tract (SHIME) model. The matrix degradation was observed with respect to the exposure time in the different sections of the SHIME: stomach after 2 hrs (Figure 3A), small intestine after 6 hrs (Figure 3B), ascending colon after 6 hrs. (Figure 3C) and ascending colon after 12 hrs (Figure 3D).
Figure 4A, 4B and 4C are images obtained by scanning electron microscopy of a FAX covalent microsphere after the *in vitro* degradation process by colonic microflora. One can observe the cavities produced by the effect of matrix degradation associated with the action of the microbiota. 350 x magnification.
Figure 5A and 5B are *In vivo* evaluation of the FAX covalent matrices. Figure 5A shows the reduction in the blood glucose levels, Figure 5B shows the Rat serum insulin concentration.

### DETAILED DESCRIPTION OF THE INVENTION

This invention relates to a covalent matrix from gel-forming feruloylated arabinoxylans, which is biodegradable and allows oral insulin administration targeting the colon. As used herein, the term "matrix" refers to a FAX gel crosslinked by covalent bonds in the shape of particles (preferably sphere or capsule) with average sizes on the order of nanometers (e.g., nanospheres; 10-999 nm, preferably from 100 to 500 nm), microns (e.g., microspheres and microcapsules; 1-999 µm, preferably from 250 to 550 µm) or millimeters (e.g., beads; 1-999 mm, preferably from 1 to 9 mm). The average size of these systems can be measured by standard procedures known to the experts in the field and that is described, for example, in the experimental portion below. The average particle size is mainly influenced by the gelling ability of the FAX and also by the conditions of the particle formation (FAX concentration, crosslinking agent concentration and ratio between them).

By the term "FAX", we are referring to feruloylated arabinoxylans, also known as pentosans, non-starch or hemicellulose polysaccharides. The FAXs are a naturally occurring polymer comprised of a linear chain of units of β-(1→4)-D-xylopyranose with branching of α-L-arabinofuranoses. Given that the arabinose is the main substituent of the xylose chain, the level of branching can be determined through the arabinose-xylose (A/X) ratio. Usually, the degree of A/X is in a range from 0.36 to 0.85, preferably from 0.6 to 0.85. A particular feature of the AXF structure is the presence of ferulic acid residues, covalently linked by ester bonds to the arabinose, which according to the preferred embodiment of this invention may vary between 0.02 and 20 µg/mg of FAX. The capability for gelation and the gel properties of the FAXs, depends on the structural characteristics of the FAXs such as molecular weight, the A/X ratio and the ferulic acid content. The FAXs used in the preparation of the FAX matrices of this invention have a molecular weight between 60 and 2000 kDa, preferably between 100 and 600 kDa, and the A/X ranges and the AXF content described above. The FAXs used are extracted from corn, but wheat, rice, barley, rye, triticale, banana, bamboo and other plant tissues reported to be a source for the feruloylated arabinoxylans may also be used as a source.

In accordance with claim 1, a process for preparing particles of FAX described as previously defined, that includes: (a) preparing an aqueous solution of feruloylated arabinoxylans, (b) preparation of an aqueous solution of the crosslinking agent, (c) preparing an aqueous solution of insulin, (d) preparing insulin-containing particles covalently crosslinked. Below are listed each of the steps to carry out this invention.

### (a) Aqueous solution of FAX

As an initial part of the process of this invention, a step to solubilize the FAX is carried out at a concentration of between 1 and 10% (w/v), preferably between 4 and 6% (w/v) in an aqueous solution with a pH of between 3 and 6, preferably 5, using mild agitation (60-90 rpm) for 2 to 4 hours at room temperature (20-25°C). This step allows FAX chains to be crosslinked to form the gel. To reduce the pH, a sodium acetate buffer solution of 0.5 M is used having a pH of between 3 and 6. A sodium citrate buffer, 0.5 M solution, having a pH of between 3 and 6 or dilute hydrochloric acid can also be used.

### (b) aqueous solution of the crosslinking agent

The second step of the invention is the preparation of a solution of the crosslinking agent. As used herein, the term "crosslinking agent" refers to all Trametes versicolor laccase enzymes and of other origin (1,2-benzenediol: oxygen oxidoreductase), with similar catalytic properties. A variant of the crosslinking agent is the use of peroxidases and manganese peroxidases. The crosslinking agent solution is prepared by a catalytic activity of between 30 to 1.67 nkat/µL, preferably from between 16.64 and 5 nkat/µL in an aqueous solution of 0.1M sodium acetate at a pH between 3 to 6, preferably 5. To reduce the pH, a sodium acetate buffer, 0.5 M solution, is used having a pH of between 3 and 6. A sodium citrate buffer, 0.5 M solution, having a pH of between 3 and 6 or dilute hydrochloric acid can also be used. The crosslinking agent allows crosslinking of the FAX chains such that a mesh is formed between which the insulin or a biologically active molecule may be inserted and subsequently released.

### (c) Aqueous solution of the insulin

As used herein, the term "insulin" refers to human recombinant insulin and insulin analogues that have similar physicochemical properties and biological activity. The insulin solution is prepared by the aggregation with glutamic acid. Glutamic acid can form aggregates of insulin-glutamic acid of a smaller size which are reversible to a pH greater than 6. The insulin having a ratio of 0.06 to 0.25 insulin/FAX (w/w), is dissolved in a dilute hydrochloric acid solution, preferably at a concentration of 0.025M (15% of total volume) and subsequently, a solution of glutamic acid, 40 to 55 M, is added (85% of total volume), with constant agitation (60-100 rpm) for 15 to 30 min at room temperature (20-25 ° C).

### (d) Preparing the FAX particles with insulin

The fourth step of this invention is the manufacture of FAX matrices loaded with insulin, which can be performed by the following methods: 1) By manual extrusion. The insulin is dissolved directly in the FAX solution, the crosslinking solution is subsequently added after the mixture is extruded onto a hydrophobic bed and left to stand until maturation of the particle matrix is achieved; 2) by the electrospraying using a triaxial system. The triaxial syringe consists of three stainless steel capillary with an outside and inside diameter (OD and ID) that varies between 0.4-0.5 mm of (ID)/0.7-0.8 mm (OD) for the capillary A, 1-1.3 mm (ID)/1.6-1.8 mm (OD) for the capillary B, 2.3-2.5 mm (ID)/3-3.2 mm (OD) for the capillary C; preferably 0.4 mm (ID)/0.711 mm (OD) for capillary A, 1.19 mm (ID)/1.65 mm (OD) for capillary B, and d 2.39 mm (ID)/3.05 mm (OD) for the capillary C. The dissolutions of steps c, a, b is injected into capillaries A, B and C, respectively, using syringe pumps at a constant flow velocity. The flow rate of the injection of the insulin solution, FAX and enzyme are 1, 1 and 2 mL/min, respectively. The distance between the needle tip and the hydrophobic liquid varies from 1 to 5 cm, preferably 5 cm. The agitation rate of the hydrophobic liquid ranges from 100 to 1000 rpm, preferably between 600 to 800 rpm. After the preparation of the matrices, the hydrophobic liquid with the matrices were shaken at a speed lower than 300 rpm for a period of time between 3 to 6 hours at 4°C, allowing solidification of the matrices. After this time, the matrices are recovered by filtration and washed three consecutive times with vegetable oil. As used in this invention, the term "hydrophobic bed" refers to the mineral oil or oily substance with similar physiochemical characteristics (e.g., the density).

Next, some illustrative examples that manifest the features and advantages of the invention are described.

### EXAMPLES

### Example 1 Formation and characterization of the covalent FAX matrices with insulin

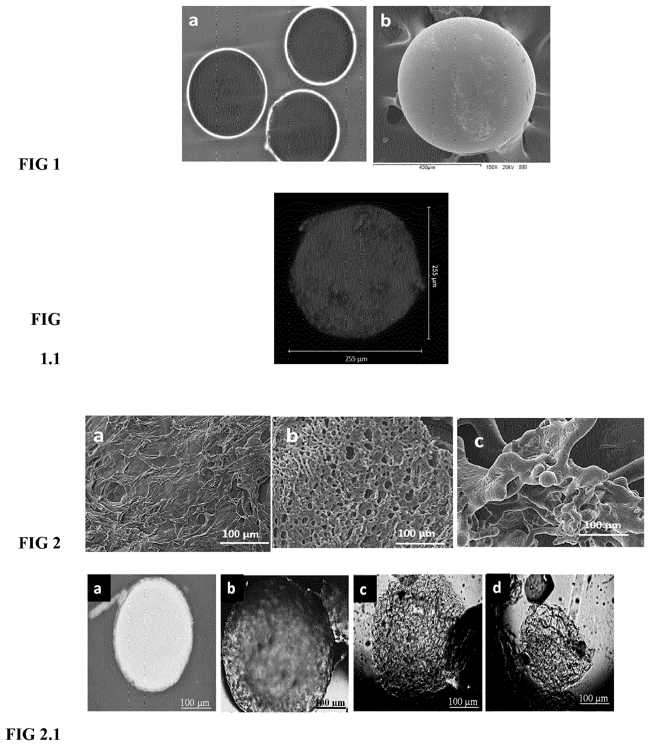

The formation of the FAX matrices with insulin are prepared according to the process described in this invention, which is mainly based on the gel times of the FAX. The mixture of the FAX with the crosslinked enzyme is extruded on a hydrophobic bed, to then leave the sphere to rest until maturation of the matrix is achieved. To decrease the size of the sphere, it was found that the concentration of the FAXs in the solution, the rate of agitation and the density of the hydrophobic bed are very important. The 6% w/v covalent FAX matrices were prepared with insulin at a 0.06 ratio of insulin/AX (w/w). The optical micrographs of the FAX matrices showed a spherical shape without aggregation (Figure 1A). The 6% FAX microspheres had a distribution of 386 µm in size with a range of 300-549 µm. Using scanning electron microscopy, it was possible to observe the microstructure of the FAX matrix (Figure 1B). The stability of the secondary structure of the insulin was studied by circular dichroism (CD). The CD spectra of insulin released from the FAX microspheres were similar to the CD spectrum of insulin that was not trapped or native. The distribution and morphology of the insulin in the 6% FAX microspheres was also evaluated by laser scanning confocal microscopy (Figure 2). The insulin loaded microspheres had a spherical morphology with an average size of 335 ± 45 µm, these characteristics were similar to the MBAX microspheres without insulin.

### Example 2 In vitro evaluation of covalent FAX microcapsules in a simulator of the human intestinal microbial ecosystem.

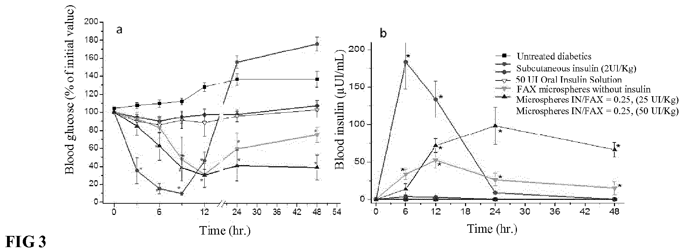

Tests were conducted on an *in vitro* model simulating the human intestinal microbial ecosystem (SHIME) in order to evaluate the degradation of the covalent FAX matrix in the region of the colon, as well as the insulin release. The three regions corresponding to the colon in the SHIME were inoculated with human feces from healthy adult volunteers aged 27 that had not used antibiotics for more than 1 year. After the inoculation, the different microbial markers, pH, temperature and time of retention were monitored for a period of 2 weeks. After this period, FAX microcapsules were administered as the principal source of carbon in the SHIME for three weeks. During this period, the principal bacterial markers and the production of short chain fatty acids were quantified. 70% of the FAX microcapsules were degraded in the ascending colon and the rest in the transverse colon. Complete fermentation of the microcapsules was carried out in the descending colon where increased production of acetic, propionic and butyric acid was recorded. In the three regions of the colon, fermentation of the FAX microcapsules significantly increased the populations of beneficial bacteria (*Bifidobacterium spp.* and *Lactobacillus spp.*) and the bacterial population where the principal pathogens are found (*Enterobacteriaceae)* was decreased. Furthermore, insulin charged microcapsules were administered during this period. In Figure 3A, 3B, 3C and 3D, microcapsules recovered from the different regions of the SHIME are shown, where it can be seen that the microcapsules are degraded to reach the ascending colon. While Figures 4A. 4B and 4C show the effect of bacterial degradation on the surface of the FAX covalent microcapsules. In these images, cavities having different sizes are seen, which increase as the time increases in which the microcapsules are exposed in the region of the colon. The results found in this *in vitro* model, show that the FAX microcapsules are able to carry insulin and resist the simulated conditions of the upper gastrointestinal system. On this route, one part of the insulin contained (~30%) is lost by diffusion and the greater part of the insulin is released in the ascending colon by matrix degradation. The main conclusion reached from these experiments is that the covalent FAX matrices are biodegradable by bacteria present in the colon and have the ability to carry and release insulin to this region by a mechanism activated by the microbiota.

### Example 3 In vitro evaluation of the insulin loaded microcapsules.

For the purpose of evaluating the ability of the FAX matrices to protect the biological activity of the insulin, tests were carried out in an *in vitro* model using diabetic rats. To carry out these tests, adult male Wistar rats were used having a body weight of between 250g and 350g, which were raised and kept in the animal experimentation Department of Research and Graduate Studies in Food of the University of Sonora, Mexico, under standard photoperiod conditions (12 hrs. light/12 hrs. dark), ambient temperature (22 ± 2°C), food and water ad libitum and sanitation schemes of their micro and macro-environment. Induction of diabetes was performed by administering a solution of streptozotocin (STZ) intraperitoneally (i.p.). Glucose levels were evaluated at 0 hours and 72 hours after administration of the STZ with the Accu-Chek glucometer (Roche Diagnostics, Thailand). Rats with glucose levels above 300 mg/dL were considered diabetic. Diabetic rats were randomly divided into six groups of six rats each for administering the different treatments. Oral administration of the treatments was performed using a buccal gastric cannula. After administration of the different treatments, blood samples were taken from the tail vein of the rats, in order to measure the levels of blood glucose and human insulin in the blood serum of the rat. These parameters were evaluated at 0, 3, 6, 9, 12, 24 and 48 hours. Glucose levels in the blood were measured with an Accu-Check blood glucose meter and the concentration of human insulin in rat serum using the ELISA technique.

In this *in vivo* model, it was found that the insulin contained in the orally administered FAX microcapsules reached biological activity in the blood circulation and that it had a hypoglycemic effect for 48 hours (Figure 3a). Furthermore, it was observed that the reduction of glucose levels with regard to time, matched the increase in the human insulin concentration in the serum of the diabetic rats (Figure 3b). The maximum time obtained in this glycemic reduction and the increase in serum insulin corresponds to the time required for the degradation of the FAX matrix by the microbiota, in agreement with that found in the *in vitro* tests performed in the SHINM. Experimental results show that the covalent FAX matrices with insulin (25 and 50 U/kg body weight) significantly reduce glycemia from 6 hours to at least 48 hours after oral administration (Figure 3a). While the insulin not contained in the covalent FAX matrices show no significant effect on the reduction of glucose levels, and its behavior is similar to that found in diabetic rats receiving no treatment or microcapsules without insulin. Regarding the results of human insulin in rat blood, constant insulin levels were observed after 12 hours with treatments with covalent FAX matrices with insulin, nevertheless, the increase of insulin from 25 to 50 U/Kg of body weight, showed a greater concentration of human insulin in the rat serum. While in treatment with insulin not contained in the FAX matrices, nonsignificant values of human insulin were found in rat serum. These results reveal that using insulin loaded covalent FAX matrices make it possible to orally deliver insulin with biological activity targeting the colon.

### BIBLIOGRAPHY

Claudia M. Berlanga-Reyes 1, Elizabeth Carvajal-Millán 2,*, Jaime Lizardi-Mendoza 2, Agustin Rascón-Chu 1, Jorge A. Marquez-Escalante 1 and Ana Luisa Martinez-López (2006) Maize Arabinoxylan Gels as Protein Delivery Matrices. Molecules 2009, 14, 1475-1482
Carvajal-Millan, E., Guilbert, S., Doublier, J.-L., and Micard, V. (2006). Arabinoxylan/protein gels: Structural, rheological and controlled release properties. Food Hydrocolloids, 20(1), 53-61.
Carvajal-Millan, E., Guilbert, S., Morel, M., and Micard, V. (2005). Impact of the structure of arabinoxylan gels on their rheological and protein transport properties. Carbohydrate Polymers, 60(4), 431-438.
Coviello, T., Matricardi, P., Marianecci, C. y Alhaique F. (2007) Polysaccharide hydrogels for modified release formulations. Journal of controlled release, 119, pp 2-24.
Gibson, G. R., Probert, H. M., Van Loo, J., Rastall, R. A, and Roberfroid, M. B. (2004) Dietary modulation of the human colonic microbiota: updating the concept of prebiotics, Nutr. Res. Review. 17: pp 259-275.
Hernández-Espinoza, A. B., Piñón-Muñiz, M. I., Rascón-Chu, A., Santana-Rodríguez, V. M., and Carvajal-Millan, E. (2012). Lycopene/arabinoxylan gels: rheological and controlled release characteristics. Molecules (Basel, Switzerland), 17(3), 2428-36.
Izydorczyk, M. S., and Biliaderis, C. G. (1995). Cereal arabinoxylans: advances in structure and physicochemical properties. Carbohydrate Polymers, 28(1), 33-48.
Iqbal, M. S., Akbar, J., Hussain, M. a., Saghir, S., & Sher, M. (2011). Evaluation of hot-water extracted arabinoxylans from ispaghula seeds as drug carriers. Carbohydrate Polymers, 83(3), 1218-1225.
Iravani, S., Fitchett, C. S., and Georget, D. M. R. (2011). Physical characterization of arabinoxylan powder and its hydrogel containing a methyl xanthine. Carbohydrate Polymers, 85(1), 201-207.
Khafagy E.S., Morishita M., Onuki Y., and Takamaya K., (2007) Current challenges in non-invasive insulin delivery systems: A comparative review. Advanced drug delivery reviews. 59, pp 1521-1541
Liu Z., Jiao, Y., Wang, Y., Zhou, C., and Zhang., Z. (2008) Polysaccharides-based nanoparticles as drug delivery systems. Advanced Drug Delivery Reviews. 60, pp 1650-1662.
Morales-Ortega, A., Carvajal-Millan, E., Brown-Bojorquez, F., Rascón-Chu, A., Torres-Chavez, P., López-Franco, Y. L., Campa-Mada, A. C. (2014). Entrapment of probiotics in water extractable arabinoxylan gels: rheological and microstructural characterization. Molecules (Basel, Switzerland), 19(3), 3628-37
Owens, D.R. (2002) New horizons-alternative routes for insulin therapy, Nature. Review Drug Discovery. 1, pp 1529-540.
Rekha MR and Sharma CP. (2013) Oral delivery of therapeutic protein/peptide for diabetes-Future perspectives. Int J Pharm, 440:48-62
Yang, L., Chu, J. S., and Fix, J. A. (2002). Colon-specific drug delivery: new approaches and in vitro/in vivo evaluation. International Journal of Pharmaceutics, 235(1-2), 1-15.

## Claims

1. A process for the formation of a covalent matrix from feruloylated arabinoxylans gelling agents carrying insulin to the colon, said matrix having a particle formation, preferably in the form of a sphere or a capsule, comprising the steps of:
(a) preparing an aqueous solution of feruloylated arabinoxylans, from feruloylated arabinoxylans with gelling capacity; that has an esterified ferulic acid to arabinoxylans content of between 0.02 and 22 µg/ mg;
(b) preparing an aqueous solution of an enzymatic crosslinked agent;
(c) preparing an aqueous solution of insulin or its analogues aggregated with glutamic acid; and
(d) production of insulin or insulin analogue-containing particles covalently crosslinked by electrospray or manual extrusion on a hydrophobic bed.

2. Process according to Claim 1, wherein the concentration of arabinoxylans in an aqueous solution has a final concentration of between 1 and 10%, preferably between 4 and 6% (w/v).

3. Process according to Claim 1, wherein the molecular weight of the feruloylated arabinoxylans has a molecular weight of between 60 and 2000 kDa, preferably between 100 and 600 kDa.

4. Process according to Claim 1, wherein the aqueous solution of the crosslinked agent has a catalytic activity of between 30 to 1.67 nkat/µL.

5. Process according to Claim 1, wherein the aqueous solution of the crosslinked agent has a catalytic activity of 16.64 to 5 nkat/µL.

6. Process according to Claim 1, wherein the crosslinked agent is selected from laccase enzymes (1,2-benzenediol: oxygen oxidoreductase), peroxidases and manganese peroxidase.

7. Process according to Claim 1, wherein the aqueous solution of insulin or insulin analogue has an insulin/arabinoxylans ratio of between 0.6 and 0.25 (w/w).

8. Process according to Claim 1, wherein the feruloylated arabinoxylan has an arabinose-xylose ratio from 0.36 to 0.85, preferably from 0.6 to 0.85.

9. A pharmaceutical composition for oral administration, comprising a covalent matrix of gelled feruloylated arabinoxylans containing insulin or an insulin analogue aggregated with glutamic acid, said matrix having a particle formation, preferably in the form of a sphere or a capsule.

10. The pharmaceutical according to Claim 9, wherein the covalent matrix with insulin is a nanoparticle with an average size of between 10 to 999 nm, preferably 100 to 500 nm.

11. The pharmaceutical according to Claim 9, wherein the covalent matrix with insulin is a nanoparticle with an average size of between 1 to 999 nm, preferably 250 to 550 nm.

12. The pharmaceutical according to Claim 9, wherein the covalent matrix with insulin is a microsphere or a microcapsule with an average size of between 1 to 999 µm.

13. The pharmaceutical according to Claim 9, which is biodegradable by the bacteria present in the region of the colon.

14. A pharmaceutical composition according to Claim 9, for use in carrying insulin to the colon.

## Patentansprüche

1. Ein Verfahren zur Bildung einer kovalenten Matrix aus feruloylierten Arabinoxylanen-Geliermitteln, die Insulin zum Dickdarm transportiert, wobei die Matrix eine Partikelgestaltung aufweist, vorzugsweise in Form einer Kugel oder einer Kapsel, umfassend die Schritte von:
(a) Herstellen einer wässrigen Lösung von feruloylierten Arabinoxylanen, aus feruloylierten Arabinoxylanen mit Gelierfähigkeit; die ein veresterte Ferulasäure zu Arabinoxylanen-Gehalt von zwischen 0,02 und 22 µg/ mg aufweist;
(b) Herstellen einer wässrigen Lösung eines enzymatischen Vernetzungsmittels;
(c) Herstellen einer wässrigen Lösung von Insulin oder seinen Analoga, die mit Glutaminsäure aggregiert sind; und
(d) Produktion von insulin- oder insulinanalogonhaltigen Partikeln, die durch Elektrospray oder manuelle Extrusion auf einem hydrophoben Bett kovalent quervernetzt sind.

2. Verfahren nach Anspruch 1, wobei die Konzentration von Arabinoxylanen in einer wässrigen Lösung eine Endkonzentration von zwischen 1 und 10%, vorzugsweise zwischen 4 und 6% (w/v) aufweist.

3. Verfahren nach Anspruch 1, wobei das Molekulargewicht der feruloylierten Arabinoxylane ein Molekulargewicht von zwischen 60 und 2000 kDa, vorzugsweise zwischen 100 und 600 kDa aufweist.

4. Verfahren nach Anspruch 1, wobei die wässrige Lösung des Vernetzungsmittels eine katalytische Aktivität von zwischen 30 und 1,67 nkat/µL aufweist.

5. Verfahren nach Anspruch 1, wobei die wässrige Lösung des Vernetzungsmittels eine katalytische Aktivität von 16,64 bis 5 nkat/µL aufweist.

6. Verfahren nach Anspruch 1, wobei das Vernetzungsmittel ausgewählt ist aus Laccase-Enzymen (1,2- Benzoldiol: Sauerstoff-Oxidoreduktase), Peroxidasen und Mangan-Peroxidase.

7. Verfahren nach Anspruch 1, wobei die wässrige Lösung von Insulin oder Insulinanalogon ein Insulin/Arabinoxylane-Verhältnis von zwischen 0,6 und 0,25 (w/w) aufweist.

8. Verfahren nach Anspruch 1, wobei das feruloylierte Arabinoxylan ein Arabinose-Xylose-Verhältnis von 0,36 bis 0,85, vorzugsweise von 0,6 bis 0,85 aufweist.

9. Eine pharmazeutische Zusammensetzung zur oralen Verabreichung, umfassend eine kovalente Matrix aus gelierten feruloylierten Arabinoxylanen, die Insulin oder ein Insulinanalogon, das mit Glutaminsäure aggregiert ist, enthält, wobei die Matrix eine Partikelgestaltung aufweist, vorzugsweise in Form einer Kugel oder einer Kapsel.

10. Das Pharmazeutikum nach Anspruch 9, wobei die kovalente Matrix mit Insulin ein Nanopartikel mit einer durchschnittlichen Größe zwischen 10 bis 999 nm, vorzugsweise 100 bis 500 nm ist.

11. Das Pharmazeutikum nach Anspruch 9, wobei die kovalente Matrix mit Insulin ein Nanopartikel mit einer durchschnittlichen Größe zwischen 1 bis 999 nm, vorzugsweise 250 bis 550 nm ist.

12. Das Pharmazeutikum nach Anspruch 9, wobei die kovalente Matrix mit Insulin eine Mikrokugel oder eine Mikrokapsel mit einer durchschnittlichen Größe zwischen 1 bis 999 µm ist.

13. Das Pharmazeutikum nach Anspruch 9, das durch die im Bereich des Dickdarms vorhandenen Bakterien biologisch abbaubar ist.

14. Eine pharmazeutische Zusammensetzung nach Anspruch 9, zur Verwendung beim Transport von Insulin zum Dickdarm.

## Revendications

1. Procédé de formation d'une matrice covalente à partir d'agents gélifiants d'arabinoxylanes féruloylés transportant de l'insuline jusqu'au côlon, ladite matrice ayant une formation de particule, de préférence sous la forme d'une sphère ou d'une capsule, comprenant les étapes de :
(a) préparation d'une solution aqueuse d'arabinoxylanes féruloylés, à partir d'arabinoxylanes féruloylés ayant une capacité gélifiante ; qui présente une teneur en acide férulique estérifié par rapport aux arabinoxylanes entre 0,02 et 22 µg/mg ;
(b) préparation d'une solution aqueuse d'un agent réticulé enzymatique ;
(c) préparation d'une solution aqueuse d'insuline ou de ses analogues agrégée avec de l'acide glutamique ; et
(d) production de particules contenant de l'insuline ou des analogues d'insuline réticulées de manière covalente par électronébullisation ou extrusion manuelle sur un lit hydrophobe.

2. Procédé selon la revendication 1, dans lequel la concentration d'arabinoxylanes dans une solution aqueuse présente une concentration finale entre 1 et 10 %, de préférence entre 4 et 6 % (w/v).

3. Procédé selon la revendication 1, dans lequel le poids moléculaire des arabinoxylanes féruloylés présente un poids moléculaire entre 60 et 2000 kDa, de préférence entre 100 et 600 kDa.

4. Procédé selon la revendication 1, dans lequel la solution aqueuse de l'agent réticulé présente une activité catalytique de 30 à 1,67 nkat/µL.

5. Procédé selon la revendication 1, dans lequel la solution aqueuse de l'agent réticulé présente une activité catalytique de 16,64 à 5 nkat/µL.

6. Procédé selon la revendication 1, dans lequel l'agent réticulé est sélectionné parmi des enzymes laccase (1,2-benzènediol : oxygène oxydoréductase), peroxydases et manganèse peroxydase.

7. Procédé selon la revendication 1, dans lequel la solution aqueuse d'insuline ou d'analogues d'insuline présente un rapport insuline/arabinoxylanes entre 0,6 et 0,25 (w/w).

8. Procédé selon la revendication 1, dans lequel l'arabinoxylane féruloylé présente un rapport arabinose-xylose de 0,36 à 0,85, de préférence de 0,6 à 0,85.

9. Composition pharmaceutique pour administration orale, comprenant une matrice covalente d'arabinoxylanes féruloylés gélifiés contenant de l'insuline ou un analogue d'insuline aggrégé avec de l'acide glutamique, ladite matrice présentant une formation de particule, de préférence sous la forme d'une sphère ou d'une capsule.

10. Composition pharmaceutique selon la revendication 9, dans laquelle la matrice covalente avec insuline est une nanoparticule ayant une taille moyenne de 10 à 999 nm, de préférence de 100 à 500 nm.

11. Composition pharmaceutique selon la revendication 9, dans laquelle la matrice covalente avec insuline est une nanoparticule ayant une taille moyenne de 1 à 999 nm, de préférence de 250 à 550 nm.

12. Composition pharmaceutique selon la revendication 9, dans laquelle la matrice covalente avec insuline est une microsphère ou une microcapsule ayant une taille moyenne de 1 à 999 nm.

13. Composition pharmaceutique selon la revendication 9, qui est biodégradable par les bactéries présentes dans la région du côlon.

14. Composition pharmaceutique selon la revendication 9, pour son utilisation dans le transport d'insuline jusqu'au côlon.
